# EUROPEAN PATENT SPECIFICATION

(11) **EP 2 902 783 B1**
(45) Date of publication and mention of the grant of the patent: **30.08.2017**
(21) Application number: 13841646.6
(22) Date of filing: 30.09.2013
(51) Int. Cl.: G01N 33/543, G01N 33/558

(54) **ADDITIVE FOR MEASURING DILUTED SAMPLE IN NON-DILUTION-TYPE IMMUNOCHROMATOGRAPHIC METHOD REAGENT**
ADDITIV ZUR MESSUNG EINER VERDÜNNTEN PROBE IN EINEM REAGENS EINER IMMUNCHROMATOGRAFISCHEN METHODE OHNE VERDÜNNUNG
ADDITIF PERMETTANT DE MESURER UN ÉCHANTILLON DILUÉ AU MOYEN D'UN RÉACTIF DANS LE CADRE D'UN PROCÉDÉ IMMUNOCHROMATOGRAPHIQUE SANS DILUTION

(30) Priority: 28.09.2012 JP 2012218560
(43) Date of publication of application: 05.08.2015
(73) Proprietor: Sekisui Medical Co., Ltd., Tokyo 103-0027 (JP)
(72) Inventor: YOSHIDA, Mayumi, Chuo-ku Tokyo 103 0027 (JP); MORITA, Motoki, Chuo-ku Tokyo 103 0027 (JP); YAMAMOTO, Mitsuaki, Chuo-ku Tokyo 103 0027 (JP)
(74) Representative: Hoffmann Eitle
(86) International application number: PCT/JP2013/076550
(87) International publication number: WO 2014/051141

(56) References cited:
- EP-A1- 0 564 880
- WO-A1-2013/130002
- CA-A1- 2 794 721
- JP-A- S5 933 224
- JP-A- H04 503 666
- JP-A- H10 185 920
- JP-A- H11 153 600
- JP-A- 2004 233 127
- JP-A- 2007 248 073
- JP-A- 2009 229 343
- JP-A- 2011 010 581
- JP-A- 2012 159 440
- US-A1- 2006 127 886
- LAGNEAU F ET AL: "Fluid therapy directly interferes with immunoassay for cardiac troponin I", INTENSIVE CARE MEDICINE 1999 DE, vol. 25, no. 6, 1999, pages 625-627, XP002756263, ISSN: 0342-4642

## Description

### TECHNICAL FIELD

The present invention relates to an immunochromatographic method.

### BACKGROUND ART

In the field of clinical examination, a measurement reagent using an immunochromatographic method which requires simple operation and a short time for measurement and enables measurement with a small inexpensive device even in the case of quantitation is extremely widespread. In order to further improve simplicity and rapidness, the demand is increasing for the development of an immunochromatographic method allowing direct use of a blood specimen (whole blood, serum, or plasma) as a sample without pretreatment such as dilution.

However, even in the case of the immunochromatographic method with a measurement system designed in such a way that a blood specimen can directly be used as a sample, if the concentration of the analyte in the blood specimen is high enough to exceed the upper limit of quantitation of the measurement system, a diluted sample of the blood specimen must be used. However, with regard to the case of directly using a blood specimen as a sample and the case of using a diluted blood specimen as a sample, the behavior of the both samples in the measurement system has never been sufficiently studied.

Patent Document 1 discloses that a nonionic water-soluble polymer with the weight-average molecular weight of 2000 to 9000 is used as a reaction accelerator acting as an additive of specimen diluent in an immunochromatographic method. It is described in this document that if the weight-average molecular weight is greater than 9000 and the concentration of the nonionic water-soluble polymer in a tested specimen is increased to acquire a sufficient reaction-accelerating effect, deterioration in spreadability of a tested solution may elongate the time required for achieving constant color development in the detection site or variations may occur in detection intensity of labeled objects among test strips.

### CITATION LIST

### PATENT LITERATURE

Patent Document 1: Japanese Laid-Open Patent Publication No. 2004-233127

### SUMMARY OF INVENTION

### TECHNICAL PROBLEM

It is an object of the present invention to provide an immunoassay method of reducing deviations from the theoretical value (hereinafter also referred to as a/the reference value) caused when a blood specimen (whole blood, serum, or plasma) is diluted and used as a sample in an immunochromatographic method designed to use a blood specimen directly as a sample without pretreatment such as dilution, and it is also an object of the present invention to provide a reagent used in this method.

### SOLUTION TO PROBLEM

The present inventors have experienced that, in an immunochromatographic method designed to use a blood specimen (whole blood, serum, or plasma) directly as a sample (hereinafter also referred to as a non-dilution method), the measurement value of an analyte in a sample may deviate from the theoretical value when the analyte is at high concentration exceeding the upper limit of quantitation of the measurement system and the blood specimen is diluted and used as a sample. As a result of intensive studies for a method of reducing this deviation, the present inventors have found that, when measurement is performed in the presence of hydroxyethyl starch at the time of measurement of a diluted blood specimen in a non-dilution method, the deviation from the theoretical value can be reduced even if a diluted blood specimen is used as a sample for the measurement, thereby completing the present invention.

The present invention has the following configuration.
[1] A method of performing immunochromatography of a blood specimen using an immunochromatographic device equipped with (1) a sample pad and (2) a membrane with an immobilized component capable of specifically binding to an analyte arranged in the order of (1) and (2) from upstream, wherein hydroxyethyl starch is present in the measurement system.
[2] The method of performing immunochromatography of [1], wherein the blood specimen is whole blood, serum, or plasma.
[3] The method of [1] or [2], wherein the immunochromatographic device further comprises (3) a conjugate pad arranged in the order of (1), (3), and (2) from upstream.
[4] The method of performing immunochromatography of [1] to [3], wherein a means of causing hydroxyethyl starch to be present in the measurement system is to contain hydroxyethyl starch in any one or more of (1) the sample pad, (2) the membrane with an immobilized component capable of specifically binding to an analyte, and (3) the conjugate pad, and (5) a blood specimen diluent.
[5] A device for immunochromatography, wherein an immunochromatographic device comprises (1) a sample pad and (2) a membrane on which a component capable of specifically binding to an analyte is immobilized, arranged in the order of (1) and (2) from upstream and hydroxyethyl starch is contained in one or more of (1) and (2).
[6] The device for immunochromatography, wherein the immunochromatographic device further comprises (3) a conjugate pad arranged in the order of (1), (3), and (2) from upstream, and wherein hydroxyethyl starch is contained in one or more of (1) to (3).
[7] Use of a blood specimen diluent for immunochromatography, the blood specimen diluent comprising hydroxyethyl starch.

### ADVANTAGEOUS EFFECTS OF INVENTION

The immunochromatographic method provided by the present invention can suppress the deviation of measurement values from the theoretical value and prevent the reduction of accuracy even if a diluted blood specimen is used as a sample for measurement in a non-dilution method.

Therefore, even if an analyte in a sample is at high concentration exceeding the upper limit of quantitation of the measurement system and a diluted blood specimen is used as a sample, accurate measurement can be performed by using the immunochromatographic method of the present invention.

### BRIEF DESCRIPTION OF DRAWINGS

[Fig. 1] Fig. 1 is a diagram showing one form of an immunochromatographic device of the present invention.
[Fig. 2] Fig. 2 is a diagram showing the correlations between the measurement result of a reference method and either the measurement result of Example 3 or the measurement result of Comparison Example 2.

### DESCRIPTION OF EMBODIMENTS

Hydroxyethyl starch (chemical name: Starch, 2-hydroxyethyl ether, hereinafter also referred to as HES) used in the present invention is a sugar compound of a high polymer that can be acquired by hydrolysis of amylopectin, which is a highly branched cornstarch component, followed by hydroxyethylation. This sugar compound has a glycoside bond (α-1,4-glycoside bond) between C4 and C1 of glucose molecules to form a main chain and also has a glycoside bond (α-1,6-glycoside bond) between C6 and C1 to form a branch chain. A hydroxyl group of C2 or C6 of glucose molecules is substituted with a hydroxyethyl group.

The property of HES is represented by molecular weight, the degree of substitution with hydroxyethyl group, the degree of dispersion (the extent of molecular weight distribution), the C2/C6 ratio (proportion between C2 and C6 substituted with hydroxyethyl group), and the like, individually or in combination with each other. For example, when the molecular weight is 70,000 and the degree of substitution with hydroxyethyl group is 0.5, the property may be represented by "HES70000/0.5", etc.

Although HES can be manufactured from cornstarch in the usual manner, HES-containing transfusion solution is also usable that is clinically used as plasma substitute/extracorporeal circulation diluent. Commercially available HES-containing transfusion solutions include HESPANDER (registered trademark) fluid solution and SALINHES (registered trademark) fluid solution 6 % (both manufactured and sold by Fresenius Kabi Japan; weight-average molecular weight: about 70,000, degree of substitution with hydroxyethyl group: 0.50 to 0.55). The following HES formulations can additionally be used individually or in combination with each other: Hetastarch (weight-average molecular weight: about 670,000, degree of substitution with hydroxyethyl group: 0.75); Pentastarch (weight-average molecular weight: about 260,000, degree of substitution with hydroxyethyl group: 0.5); Elohes (weight-average molecular weight: about 200,000, degree of substitution with hydroxyethyl group: 0.62); Primmer (weight-average molecular weight: about 200,000, degree of substitution with hydroxyethyl group: 0.5); and Voluven (weight-average molecular weight: about 130,000, degree of substitution with hydroxyethyl group: 0.4).

Methods of causing HES to be present in a measurement system in an immunochromatographic method of the present invention include (A) a method in which HES is mixed with a sample in a solution state in advance and (B) a method in which HES is contained in one or more members of an immunochromatographic device.

The method of (A) may be a method in which HES is contained in a blood specimen diluent. In this case, the concentration of HES in the diluent is preferably 1.0 wt. % to 2.5 wt. %. The mixing ratio (dilution rate) between the sample and the diluent can experimentally be set in consideration of the concentration of the analyte in the sample, the upper limit of quantitation of the immunochromatographic method, the amount of sample added to the sample pad, etc. For example, if BNP in plasma is the analyte and the lower and the upper limits of quantitation of the immunochromatographic method are 10 pg/mL and 800 pg/mL, respectively, 10-fold dilution is preferable.

HES is preferably dissolved in a suitable buffer solution before use. Any buffer agents used in immunochromatographic methods are usable, including a phosphate buffer solution, a glycine buffer solution, a Tris buffer solution, a boric-acid buffer solution, and a citric-acid buffer solution, as long as no adverse effect is given to the performance, e.g., stability and sensitivity of antigens and antibodies, of the immunochromatographic method. In this case, a preferred pH range is 5.5 to 9.0. The same applies to additives (nonspecific reaction suppressants, sensitizers, stabilizers, and preservatives) used in an immunochromatographic method.

The method of (B) will hereinafter be described in association with description of the immunochromatographic device.

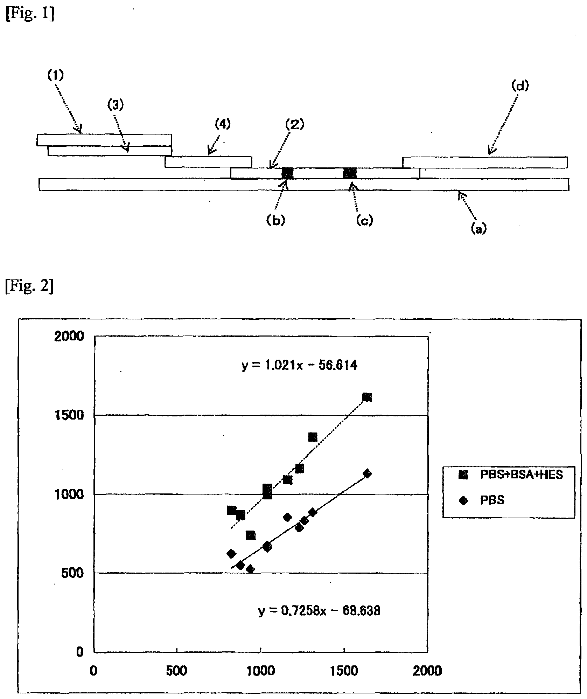

The immunochromatographic device of the present invention has (1) a sample pad and (2) a membrane on which a component capable of specifically binding to an analyte is immobilized, arranged in the order of (1) and (2) from upstream. Hereinafter, assuming that the analyte is an antigen and the component capable of specifically binding to the analyte is a specific antibody to the antigen (hereinafter also referred to as a specific antibody), the method will be described, with reference to Fig. 1, by taking as an example a sandwich method in which a complex of antigen and specific antibody formed by using two or more specific antibodies is detected. The term "upstream" in this description is defined such that the sample added to the sample pad spreads through the membrane in the downstream direction.
(1) A sample pad is a member for receiving a sample possibly containing an analyte (antigen) and is preferably made of material such as glass fiber.
(2) A membrane on which a component (specific antibody to an antigen (b)) capable of specifically binding to the analyte is immobilized, can be acquired by applying in line-shape a solution containing the specific antibody. The linearly applied specific antibody assumes a role of capturing and concentrating the antigen on the membrane by forming with the antigen the complex of specific antibody and antigen. Nitrocellulose may preferably be used as the material for the membrane. The specific antibody may be an immunoglobulin molecule itself or may be a fragment having binding ability to the antigen such as F(ab')₂, for example. The antibody may be a polyclonal antibody or a monoclonal antibody. The antibody is not limited by the method of acquisition regardless of the usage of genetic recombination techniques, the utilization of DNA immunization methods, and the like.

In order to detect the complex of antigen and specific antibody captured and concentrated on the membrane, a specific antibody labeled with a labeling substance is necessary and is called a conjugate. The labeling substance may preferably be an enzyme (such as peroxidase), chromogenic or luminous pigment (such as fluorescein), metal colloid (such as colloidal gold), and colored particulates (such as color/colored latex).

If the conjugate is used in a solution state, the conjugate can be mixed with the sample in advance and added to the sample pad, or can be added to the sample pad after the sample is added to the sample pad (if the conjugate solution contains HES, this is included in the embodiment of (A) described above). If the immunochromatographic device has (3) a conjugate pad, the conjugate may be impregnated in the conjugate pad to make up the immunochromatographic device. In an embodiment with (3) a conjugate pad, a complex of the antigen and the conjugate is formed before the antigen in the sample is captured and concentrated on the membrane. Glass fiber is preferably used as the material for the conjugate pad. If the sample pad also has the function of the conjugate pad, a single member is considered as (1) and (3).

The immunochromatographic device of the present invention may have (4) a blood cell separation pad for the purpose of capturing blood cells in whole blood. If (4) a blood cell separation pad is included, the members are preferably arranged in, but not limited to, the order of (1), (3), and (4) from upstream. The material of the blood cell separation pad is preferably polysulphone. The method (B) described above is an embodiment in which HES is contained in one or more members of the immunochromatographic device and therefore may be any embodiment in which HES is contained in any one or more of (1), (2), (3), and (4) and, particularly, HES is more preferably contained in (1). Also, if HES is contained in any one or more of (1), (2), (3), and (4), liquid HES may be impregnated in each of the members or, HES may be impregnated and dried such that the HES ingredient is contained in a dry state in each of the members. If HES is contained in any one or more of (1), (2), (3), and (4), the concentration thereof may experimentally be set in consideration of the volumes of the members of the immunochromatographic device as well as the preferred concentration and the mixing ratio to the sample in (A) the method in which HES is contained in the blood specimen diluent described above.

In the method of manufacturing immunochromatographic device, any techniques (e.g., blocking) employed in an immunochromatographic method can be used as long as no adverse effect is given to the performance of the immunochromatographic method, e.g., stability of antigens and antibodies and sensitivity of the method. The same applies to additives (nonspecific reaction suppressants, sensitizers, moisturizers, stabilizers, and preservatives) used in an immunochromatographic method. As shown in Fig. 1, an immunochromatographic device may be configured such that a membrane is attached to a plastic adhesive sheet (a), such that a control antibody (c) is applied to the membrane for checking whether the sample-loading operation is accomplished, and such that an absorption pad (d) is included for absorbing the liquid component derived from the sample in the most downstream portion of the membrane.

In the present invention, the analyte is not limited as long as the analyte can be used in an antigen-antibody reaction, and may preferably be those with high demand for being measured in whole blood in clinical test, such as C-reactive protein (CRP), human fibrinogen, D-dimer, and BNP (human brain natriuretic peptide).

Although the method of detecting analyte with the immunochromatographic device of the present invention is not particularly limited, it is preferable to optically measure the accumulation of the conjugate captured and concentrated on the membrane, and the maximum effect can be acquired in a quantitation method using equipment detecting absorbance or reflected light intensity.

### EXAMPLES

The present invention will hereinafter specifically be described with Examples. However, the present invention is not limited to these Examples.

### [Comparison Example 1] <Confirmation of Effect on Measurement When Plasma Is Diluted and Used as a Sample>

Rapid Chip (registered trademark) BNP (manufactured by SEKISUI MEDICAL Co., Ltd.) which is to be used with undiluted plasma directly as a sample in measurement was employed as an immunochromatography reagent. Plasma was diluted ten times with saline or a 10 mmol/L phosphate buffer solution (pH 7.2) containing 150 mmol/L NaCl (hereinafter referred to as PBS) to measure the concentration of BNP in accordance with operation described in the attached document. The BNP concentration in plasma was 542 pg/mL in the measurement using MI02 Shionogi BNP (SHIONOGI & CO., Ltd) and this was used as a reference value for accuracy.

The BNP concentration of the sample diluted by saline was 316 pg/mL and a recovery rate relative to the reference value was 53.9 %. The BNP concentration of the sample diluted by PBS was 385 pg/mL and a recovery rate relative to the reference value was 70.9 %.

### [Example 1] <Confirmation of Effect of Additive of the Present Invention: HES - 1>

The same operation as Comparison Example 1 was performed except that plasma was diluted ten times by a PBS containing BSA or HES at concentrations indicated in Table 1. For HES, HESPANDER (registered trademark) fluid solution was used (manufactured and sold by Fresenius Kabi Japan; weight-average molecular weight: about 70,000, degree of substitution with hydroxyethyl group: 0.50 to 0.55; HES concentration of 6 wt. %). The HES concentration in Table 1 represents an actual HES concentration in a diluent.

The measurement result is shown in Table 1.

In the case of dilution with PBS containing BSA or HES, an improvement in recovery rate relative to the reference value was confirmed.

In the studied concentration range, the improving effect of HES was greater than the improving effect of BSA.

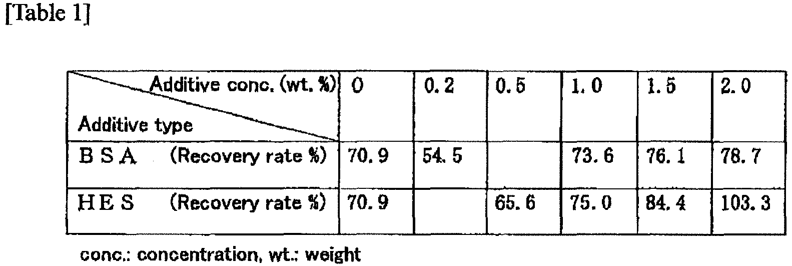

A blank indicates that the test was not conducted.

### [Example 2] <Confirmation of Effect of Additive of the Present Invention: HES - 2>

The same operation as Comparison Example 1 was performed except that plasma was diluted ten times by a PBS containing HES at concentrations indicated in Table 2 and 1.0 % BSA.

The measurement result is described in Table 2.

The recovery rate relative to the reference value was further improved by allowing HES and BSA to coexist.

**[Table 2]**

| | | | | | |
|---|---|---|---|---|---|
| HES concentration (wt. %) | 0 | 0.5 | 1.0 | 1.5 | 2.0 |
| Recovery rate (%) | 70.9 | 72.1 | 80.9 | 89.6 | 98.3 |

### [Example 3] <Confirmation of Effect of Additive of the Present Invention: HES - 3>

The same operation as Comparison Example 1 was performed except that the plasmas (nine specimens) each producing a measurement value equal to or greater than 800 pg/mL and equal to or less than 2000 pg/mL in the measurement by MI02 Shionogi BNP (SHIONOGI) were diluted ten times by PBS (Comparison Example 2) or a PBS containing 1.5 % HES and 1.0 % BSA (Example 3). The correlation with MI02 Shionogi BNP was examined.

The measurement result is shown in Fig. 2.

When a measurement was performed on whole blood diluted with a PBS containing 1.5 % HES and 1.0 % BSA (Example 3), the correlation with the reference value was improved as compared to a measurement performed on whole blood diluted with PBS (Comparison Example 2).

### INDUSTRIAL APPLICABILITY

Even if an analyte in a sample is at high concentration exceeding the upper limit of quantitation of the measurement system and a diluted blood specimen is used as a sample, accurate measurement can be performed by using the immunochromatographic method of the present invention.

### REFERENCE SIGNS LIST

(1) Sample pad
(2) Membrane
(3) Conjugate pad
(4) Blood cell separation pad
   (a) Plastic adhesive sheet
   (b) Specific antibody against antigen
   (c) Control antibody
   (d) Absorption pad

## Claims

1. An immunochromatographic method performed on a blood specimen using an immunochromatographic device equipped with (1) a sample pad and (2) a membrane on which a component capable of specifically binding to an analyte is immobilized, arranged in the order of (1) and (2) from upstream, wherein hydroxyethyl starch is present in the measurement system.

2. The immunochromatographic method according to claim 1, wherein the blood specimen is whole blood, serum, or plasma.

3. The method according to claim 1 or 2, wherein the immunochromatographic device further comprises (3) a conjugate pad arranged in the order of (1), (3), and (2) from upstream.

4. The immunochromatographic method according to any one of claims 1 to 3, wherein a means of causing hydroxyethyl starch to be present in the measurement system is to contain hydroxyethyl starch in any one or more of (1) the sample pad, (2) the membrane on which a component capable of specifically binding to an analyte is immobilized, and (3) the conjugate pad, and (5) a blood specimen diluent.

5. The immunochromatographic method according to any one of claims 1 to 4, wherein hydroxyethyl starch is contained in a blood specimen diluent and the concentration of hydroxyethyl starch in the diluent is 1.0 wt.% to 2.5 wt.%.

6. A device for immunochromatography, wherein an immunochromatographic device comprises (1) a sample pad and (2) a membrane on which a component capable of specifically binding to an analyte is immobilized, arranged in the order of (1) and (2) from upstream and that hydroxyethyl starch is contained in one or more of (1) and (2).

7. The device for immunochromatography of claim 6, wherein the immunochromatographic device further comprises (3) a conjugate pad arranged in the order of (1), (3), and (2) from upstream, and wherein hydroxyethyl starch is contained in one or more of (1) to (3).

8. Use of a blood specimen diluent for immunochromatography, the blood specimen diluent comprising hydroxyethyl starch.

## Patentansprüche

1. Immunchromatografisches Verfahren, das an einer Blutprobe unter Verwendung einer immunchromatografischen Vorrichtung durchgeführt wird, die mit (1) einem Probenpad und (2) einer Membran, auf der eine Komponente, die in der Lage ist, insbesondere mit einem Analyten zu verbinden, immobilisiert ist, ausgestattet ist, die in der Reihenfolge von (1) und (2) von stromaufwärts angeordnet sind, wobei Hydroxyethylstärke in dem Messsystem vorhanden ist.

2. Immunchromatografisches Verfahren nach Anspruch 1, wobei die Blutprobe Vollblut, Serum oder Plasma ist.

3. Verfahren nach Anspruch 1 oder 2, wobei die immunchromatografische Vorrichtung weiter umfasst (3) ein konjungiertes Pad, das in der Reihenfolge (1), (3) und (2) von stromaufwärts angeordnet ist.

4. Immunchromatografisches Verfahren nach einem der Ansprüche 1 bis 3, wobei ein Mittel zum Veranlassen, das Hydroxyethylstärke in dem Messsystem vorhanden ist, ist, dass Hydroxyethylstärke in einem oder mehreren von (1) dem Probenpad, (2) der Membran, auf der eine Komponente, die in der Lage ist, insbesondere mit einem Analyten zu verbinden, immobilisiert ist, und (3) dem konjugierten Pad und (5) einem Blutprobenverdünnungsmittel enthalten ist.

5. Immunchromatografisches Verfahren nach einem der Ansprüche 1 bis 4, wobei Hydroxyethylstärke in einem Blutprobenverdünnungsmittel enthalten ist und die Konzentration von Hydroxyethylstärke in dem Verdünnungsmittel 1,0 Gew.-% bis 2,5 Gew.-% beträgt.

6. Vorrichtung für Immunchromatografie, wobei eine Vorrichtung für Immunchromatografie umfasst (1) ein Probenpad und (2) eine Membran, auf der eine Komponente, die in der Lage ist, insbesondere mit einem Analyten zu verbinden, immobilisiert ist, die in der Reihenfolge von (1) und (2) von stromaufwärts angeordnet sind, und wobei Hydroxyethylstärke in einem oder mehreren von (1) und (2) enthalten ist.

7. Vorrichtung für Immunchromatografie nach Anspruch 6, wobei die Vorrichtung für Immunchromatografie weiter umfasst (3) ein konjungiertes Pad, das in der Reihenfolge (1), (3) und (2) von stromaufwärts angeordnet ist, und wobei Hydroxyethylstärke in einem oder mehreren von (1) bis (3) enthalten ist.

8. Verwenden eines Blutprobenverdünnungsmittels für Immunchromatografie, wobei das Blutprobenverdünnungsmittel Hydroxyethylstärke umfasst.

## Revendications

1. Procédé immunochromatographique effectué sur un spécimen de sang à l'aide d'un dispositif immunochromatographique équipé (1) d'un tampon échantillon et (2) d'une membrane sur laquelle un composant capable de se lier spécifiquement à un analyte est immobilisé, disposés dans l'ordre (1) et (2) depuis l'amont, dans lequel de l'hydroxyéthylamidon est présent dans le système de mesure.

2. Procédé immunochromatographique selon la revendication 1, dans lequel le spécimen de sang est du sang total, du sérum ou du plasma.

3. Procédé selon la revendication 1 ou 2, dans lequel le dispositif immunochromatographique comprend en outre (3) un tampon conjugué, avec un agencement dans l'ordre (1), (3) et (2) depuis l'amont.

4. Procédé immunochromatographique selon l'une quelconque des revendications 1 à 3, dans lequel un moyen pour amener de l'hydroxyéthylamidon à être présent dans le système de mesure consiste à renfermer de l'hydroxyéthylamidon dans un ou plusieurs parmi : (1) le tampon échantillon, (2) la membrane sur laquelle un composant capable de se lier spécifiquement à un analyte est immobilisé, et (3) le tampon conjugué, et (5) un diluant de spécimen de sang.

5. Procédé immunochromatographique selon l'une quelconque des revendications 1 à 4, dans lequel de l'hydroxyéthylamidon est contenu dans un diluant de spécimen de sang et la concentration d'hydroxyéthylamidon dans le diluant est de 1,0 % en poids à 2,5 % en poids.

6. Dispositif d'immunochromatographie, dans lequel un dispositif immunochromatographique comprend (1) un tampon d'échantillon et (2) une membrane sur laquelle un composant capable de se lier spécifiquement à un analyte est immobilisé, agencés dans l'ordre (1) et (2) depuis l'amont, et dans lequel de l'hydroxyéthylamidon est contenu dans un ou plusieurs de (1) et (2).

7. Dispositif d'immunochromatographie selon la revendication 6, dans lequel le dispositif immunochromatographique comprend en outre (3) un tampon conjugué, avec un agencement dans l'ordre (1), (3) et (2) depuis l'amont, et dans lequel de l'hydroxyéthylamidon est contenu dans un ou plusieurs de (1) à (3).

8. Utilisation d'un diluant de spécimen de sang pour une immunochromatographie, le diluant de spécimen de sang comprenant de l'hydroxyéthylamidon.
